(19) **Europäisches Patentamt / European Patent Office / Office européen des brevets**

(11) **EP 4 667 904 A1**

(12) **EUROPEAN PATENT APPLICATION**

(43) Date of publication:
24.12.2025 Bulletin 2025/52

(51) International Patent Classification (IPC):
*G01N 17/00* $^{(2006.01)}$

(21) Application number: 25180447.2

(52) Cooperative Patent Classification (CPC):
G01N 17/006

(22) Date of filing: 03.06.2025

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
GE KH LA MA MD TN

(30) Priority: 18.06.2024 US 202418747078

(71) Applicant: **The Boeing Company**
**Arlington, VA 22202 (US)**

(72) Inventors:
• WILLIAMS, John Dalton
Arlington, 22202 (US)
• MITCHELL, Michael F.
Arlington, 22202 (US)
• JARA, Adriana
Arlington, 22202 (US)
• KARAYAN, Ahmad I.
Arlington, 22202 (US)
• KAPILA, Vivek
Arlington, 22202 (US)
• KWAN, Kalsi
Arlington, 22202 (US)
• WILLIAMS, Kristen S.
Arlington, 22202 (US)

(74) Representative: **Boult Wade Tennant LLP**
**Salisbury Square House**
**8 Salisbury Square**
**London EC4Y 8AP (GB)**

(54) **CORROSION DETECTION SENSORS**

(57) Systems, apparatus, articles of manufacture, and methods for corrosion detection are disclosed. An example apparatus includes an adhesive to removably couple the apparatus directly to a region of a substrate; a first sensor to measure a first parameter at the region; a second sensor to measure a second parameter at the region, the first parameter and the second parameter being factors of corrosion at the region; and a power supply to provide power to the first sensor and the second sensor.

FIG. 1

EP 4 667 904 A1

**Description**

FIELD OF THE DISCLOSURE

This disclosure relates to sensors for detecting corrosion.

BACKGROUND

Metal surfaces are susceptible to corrosion. Corrosion detection is an element of condition-based maintenance of machines.

SUMMARY

Systems, apparatus, articles of manufacture, and methods for corrosion detection are disclosed. An example apparatus includes an adhesive to removably couple the apparatus directly to a region of a substrate, a first sensor to measure a first parameter at the region, and a second sensor to measure a second parameter at the region. The first parameter and the second parameter are factors of corrosion at the region. The apparatus also includes a power supply to provide power to the first sensor and the second sensor.

An example Internet of Things (IoT) device includes an impedance sensor to measure impedance of a substrate, machine-readable instructions, and at least one processor circuit to be programmed by the machine-readable instructions to determine the impedance of the substrate from the impedance sensor at multiple frequencies. The IoT device also includes a power supply to power the impedance sensor and the at least one processor circuit and an adhesive to couple the IoT device to the substrate.

An example aircraft includes a corrosion detection sensor including an impedance sensor to measure impedance at a portion of a substrate of the aircraft, first machine-readable instructions, and first programmable circuitry to be programmed by the first machine-readable instructions to wirelessly communicate data related to the impedance. The aircraft also includes a device remote from the corrosion detection sensor. The device includes second machine-readable instructions and second programmable circuitry to be programmed by the second machine-readable instructions to wirelessly access the data related to the impedance and determine a level of corrosion of the portion based on the data related to the impedance.

BRIEF DESCRIPTION OF THE DRAWINGS

FIG. 1 is an illustration of an example lap joint joining two example metal substrates and an example corrosion detection sensor.
FIG. 2 is a block diagram of an example environment in which the corrosion detection sensor of FIG. 1 and an example remote device operative to detect corrosion.
FIG. 3A is a schematic illustration of an example of the corrosion detection sensor of FIGS. 1 and 2.
FIG. 3B is a schematic illustration of an example of the impedance sensor of FIG. 2.
FIG. 4A is a schematic top view of an example interdigitated electrode (IDE).
FIG. 4B is a schematic side view of another example impedance sensor incorporating the example IDE of FIG. 4A and coupled to an example substrate.
FIG. 5 is a schematic top view of another example impedance sensor.
FIG. 6 is a schematic side view of another example corrosion detection sensor.
FIG. 7 is a block diagram of an example processing platform including programmable circuitry structured to execute, instantiate, and/or perform example machine readable instructions and/or perform example operations disclosed herein to implement elements of the corrosion detection sensor and/or the remote device of FIG. 2.

In general, the same reference numbers will be used throughout the drawing(s) and accompanying written description to refer to the same or like parts. The figures are not necessarily to scale. Instead, the thickness of the layers or regions may be enlarged in the drawings. Although the figures show layers and regions with clean lines and boundaries, some or all of these lines and/or boundaries may be idealized. In reality, the boundaries and/or lines may be unobservable, blended, and/or irregular.

DETAILED DESCRIPTION

Corrosion is the primary cause for refurbishment on bridges, ships, and buildings near salt water. Corrosion also affects metal substrates in other environments and on other structures and/or machines such as, for example, automobiles and/or aircraft, communications equipment, weapons, etc. All metal surfaces are susceptible to corrosion. Some areas may be more prone to corrosion such as, for example, areas where contaminants and/or water can permeate and/or collect. Example areas that may be more prone to corrosion include areas at joints, seams, bends, crevices, cracks, edges, apertures, fasteners, material interfaces, areas near stray current discharge, and/or weakly treated or painted surfaces. Example areas of interest may be painted, prime, treated, and/or left bare. In some examples, the substrate is a bare metal surface, a bare composite surface, a primed surface, a painted surface, a surface with a layer of sealant, a surface with a layer of a corrosion-inhibiting compound (CIC), a surface with a layer of lubricant, and/or a combination of materials and/or surface treatments. Example substrates disclosed herein may be incorporated into a vehicle (e.g., aircraft, ships, trains, land and/or motor vehicles such as a motorcycle, a car in snowy conditions etc.), a structure (e.g., railroad tracks, bridges, buildings, etc.), electrical housings located near a salt water beach, weapons, high temperature factory environments, salt processing facilities, structures near salt water, structures near or in environments of vastly different temperature oscillations, and/or any physical structure to be maintained in the presence of a short or long term corrosive environmental system. Example substrates disclosed here may be any oscillating platform (e.g., a bridge) or any static platform (e.g., building, pipe, or metal object) located in a dynamic environment where aging, ambient air conditions, and/or liquid contact cause long term electrochemical degradation.

Corrosion prevention, prediction, detection, and monitoring assist in the maintenance of infrastructure and/or machines. Current corrosion monitoring tools have limited ability to measure and/or record data at a point, a portion, a region, or a region of interest (ROI) including challenging ROIs such as, for example, lap joints. Throughout this description, the term ROI is used to refer to any area where corrosion detection is monitored. Current solutions have a significantly large size, require substantial battery power, and have limited ability to measure the details of the environment at ROIs. Where data cannot be collected at an ROI, estimates may be used. The lack of comprehensive data hinders accurate evaluation and/or prediction of corrosion potential in environments such as, for example, operational aerospace environments.

Examples disclosed herein include corrosion detection sensors that have a small size (e.g., 2-3 millimeters (mm) of thickness) that can be directly adhered to a ROI including, for example, ROIs in small and/or hard-to-reach spaces. Examples disclosed herein include battery-powered devices that consume small amounts (e.g., microwatts) of power between sample times, ensuring long-lasting operation. Examples disclosed herein provide precise and localized data collection. Such data allows for accurate evaluation and prediction of corrosion, which leads to improved maintenance strategies and increased operational efficiency.

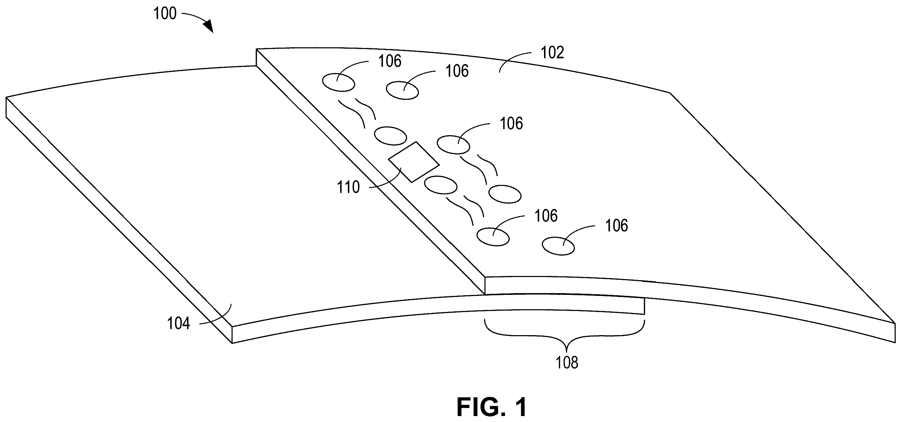

FIG. 1 is an illustration of an example substrate 100 that includes a first example panel 102 coupled to a second example panel 104 via a plurality of example fasteners 106. The fasteners 106 may include rivets, nuts, bolts, washers, screws, nails, and other types of mechanical fasteners. The substrate 100 may be incorporated into a vehicle (e.g., an aircraft), a structure (e.g., a bridge), a device (e.g., a missile), and/or other equipment or apparatus that includes metal components. In some examples, including the example of FIG. 1, the joint is a lap joint. A lap joint is a joint in which the joined members overlap. In some examples, the lap joint is an area prone to corrosion. Thus, in examples disclosed herein, the lap joint may form an example ROI 108. An example corrosion detection sensor 110 is placed in the ROI 108 to gather data to be used in the evaluation and prediction of corrosion in the ROI 108. In some examples, the corrosion detection sensor 110 is a peel-and-stick device. A liner is removed to expose an adhesive layer of the corrosion detection sensor 110. With the adhesive layer exposed, the corrosion detection sensor 110 is coupled to the substrate 100 at the ROI 108. The corrosion detection sensor 110 includes a flexible substrate that can be mounted to a flat or curved surface. In some examples, the adhesive is permanent. In some other examples, the adhesive is a releasable adhesive, and the corrosion detection sensor 110 can be removed from the substrate 100. In some examples, the adhesive is releasable and reusable, and the corrosion detection sensor 110 can be removed from the ROI 108 and placed onto another area of the substrate 100 or on a different substrate.

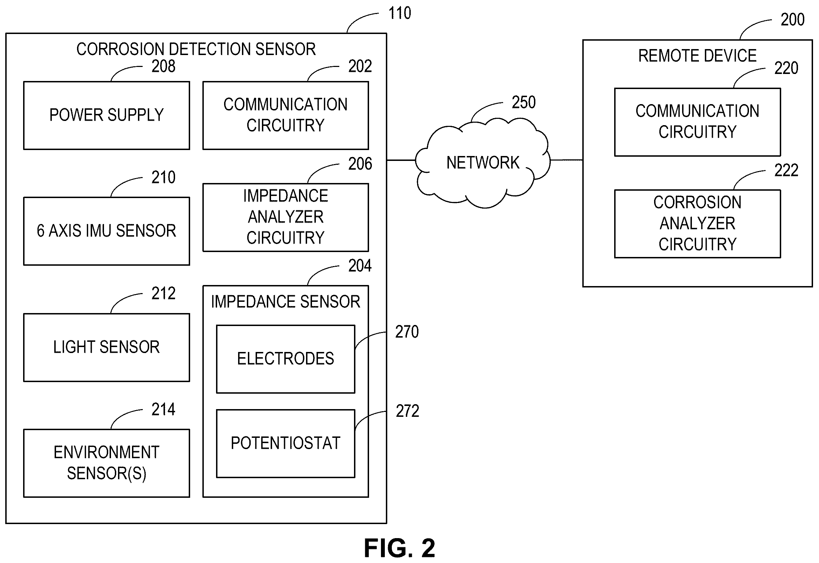

FIG. 2 is a block diagram of an example environment in which the corrosion detection sensor 110 and an example remote device 200 operate to detect corrosion. The corrosion detection sensor 110 includes example communication circuitry 202, an example impedance sensor 204, example impedance analyzer circuitry 206, an example power supply 208, and a plurality of additional sensors including, for example, an example 6-axis IMU (inertial measurement unit) sensor 210, an example light sensor 212, and one or more environment sensors 214. Example environment sensors include temperature sensors, relative humidity sensors, pH sensors, vibration sensors, mechanical strain sensors (e.g., strain gauges), gas concentration sensors, etc. In some examples, the power supply 208 is a battery such as, for example, a coin cell battery. In some examples, the power supply 208 includes a voltaic cell. In some examples, the power supply 208 includes a solar cell or photovoltaic cell.

The remote device 200 includes example communication circuitry 220 and example corrosion analyzer circuitry 222. The corrosion detection sensor 110 and the remote device 200 are communicatively coupled over an example network 250. The network 250 may be wired or wireless and supports any communication protocol including, for example, Bluetooth low energy (BLE), Wi-Fi, LTE-M, CBRS, ultra-wideband (UWB), Zigbee, radio, LoRa, 5G, next generation communication protocols, etc. In some examples, the corrosion detection sensor 110 and/or the remote device 200 are Internet of Things (IoT) devices.

The impedance sensor 204 measures electrochemical impedance. That is, impedance sensor 204 measures the resistance, capacitance, and inductance present across a frequency range recorded for the circuit at the time of each measurement sweep (measurement as a function of frequency taken at time x). Thus, electrochemical impedance spectroscopy allows for the complete understanding of electrical properties of the material interface being recorded. Changes in those electrical properties (i.e., the impedance as a function of frequency) can be used to provide a complete and definitive assessment of the corrosive response occurring at the measurement site. The impedance data and data related to other environmental factors can be used to determine if and/or why a material has changed, to measure any change in material properties, and/or prediction material change under different environmental conditions. The impedance sensor 204 includes example electrodes 270 that are coupled to a potentiostat 272. Power from the power supply 208 is provided to the impedance sensor 204, and the potentiostat 272 measures the potential or voltage difference between the two electrodes 272. The potentiostat 272 measures current response from a substrate that will be converted to impedance by the impedance analyzer circuitry 206. In some examples, the electrodes 272 are 2-3 mm printed metal electrodes. In some examples, the electrodes 272 are gold. In some other examples, the electrodes 272 are platinum, rhodium, ruthenium, osmium, iridium, palladium, or other noble metal. In some examples, the electrodes 272 are interdigitated electrodes (IDEs). In some examples, the electrodes 272 may also use other geometries to create capacitively coupled resistive sensing. In some examples, the substrate 100 also serves as an electrode.

The impedance sensor 204 is coupled to the impedance analyzer circuitry 206. In some examples, the impedance sensor 204 is wired to the impedance analyzer circuitry 206. The impedance analyzer circuitry 206 records the impedance of the electrodes 272 over a range of frequencies and over time. In some examples, the frequency range for which data is gathered and analyzed includes frequencies from about 30 Hertz (Hz) to about 100,000 Hz, or from 30 Hertz (Hz) to 100,000 Hz. In some other examples, the frequency range may include other values based on the device and/or associated work function. The impedance profile of the substrate 100 may be analyzed to assess a corrosion level of the substrate 100. The impedance sensor 204 may be used on electrochemically active substrates or passive metallic surfaces.

The corrosion detection sensor 110 also includes other sensors to gather data that may be analyzed to assess a corrosion level of the substrate 100. For example, the 6-axis IMU sensor 210 includes a 3-axis accelerometer and a 3-axis gyroscope, which measure the six degrees of freedom: roll, pitch, yaw, thrust, heave, sway. The data from the 6-axis IMU sensor 210 provides context to a corrosion measurement. An IMU provides an additional level to discern potential increases in corrosion and their rates. Mechanical vibration, strain, and stress loading can have an effect on electrochemical properties of the substate 100. For example, electrochemical voltage potential, E, can be derived from the standard cell potential, $E_0$, stoichiometric conditions, a-d and n, and chemical concentrations of all reactants and products, [A-D], involved in the corrosion process as shown in Equation (1).

$$E = E_0 - \frac{RT}{nF}lnQ' = E_0 - \frac{RT}{nF}ln\left(\left(\frac{[C]^c[D]^d}{[A]^a[B]^b}\right) + k_\sigma\left(\frac{\sigma}{\sigma_y}\right) + k_\omega\left(\frac{\omega}{\omega_o}\right) + k_{RH}\left(\frac{RH}{45\%RH}\right) + k_{UV}\left(\frac{UV}{UV_o}\right)\right)$$

Equation (1)

where R is the ideal gas constant (8.314 J/(Mol*K), F is Faraday's constant (96.485 J/(V*Mol)), and T is the temperature in Kelvin at the corrosion site. Other constants such as $k_i$ represent linear constants relating the chemical potential to the normalized i'th physical parameter. Here, $\sigma$ represents applied stress, and $\sigma_y$ represents the yield stress of the material, UV describes ultraviolet light intensity, RH represents the relative humidity, and $\omega$ represents the vibrational oscillation frequency.

In some examples, the light sensor 212 measure ultra-violet (UV) light exposure. The environment sensors 214 gather data related to additional characteristics of the environment in which the corrosion detection sensor 110 is placed including, for example, pH, vibration, mechanical strain, gas concentration, humidity, temperature, etc.

In some examples data from the corrosion detection sensor 110 is gathered over time. In some examples data from the corrosion detection sensor 110 is gathered continuously. In some examples data from the corrosion detection sensor 110 is gathered when a request is received via the communication circuitry 202 of the corrosion detection sensor 110. For example, the remote device 200 may communicate via the communication circuitry 220 of the remote device 200 to the corrosion detection sensor 110 to sample data, transfer data, and/or alter measurement and communication cadence.

The corrosion detection sensor 110 transmits data to the remote device 200. The corrosion analyzer circuitry 222 analyzes the data to assess a corrosion level and/or create a corrosion profile of the substrate 110. Different factors could be indicative a higher level of corrosion or greater likelihood of corrosion. For example, higher humidity levels, more UV light exposure, and/or higher concentration of salt in the environment may be more indicative of corrosion than, for example, lower humidity level, less UV light exposure, and/or lower concentration of salt in the environment. In addition, a higher impedance may be indicative of greater corrosion resistance. A lower impedance may be indicative of corrosion. In addition, the corrosion analyzer circuitry 222 analyzes the impedance data over time. In some examples, the corrosion analyzer circuitry 222 implements machine learning and artificial intelligence to assess and/or predict corrosion.

In some examples, the remote device 200 is located at another area of the substrate 100. In some examples, the remote device 200 is located elsewhere in the same housing as the corrosion detection sensor 110. For example, the corrosion detection sensor 110 may be placed on a surface of an aircraft, and the remote device 200 is located within the aircraft. In some examples, the remote device is in another facility separate from the corrosion Detection sensor 110. In some examples, the remote device 200 is cloud-based, edge-based, etc.

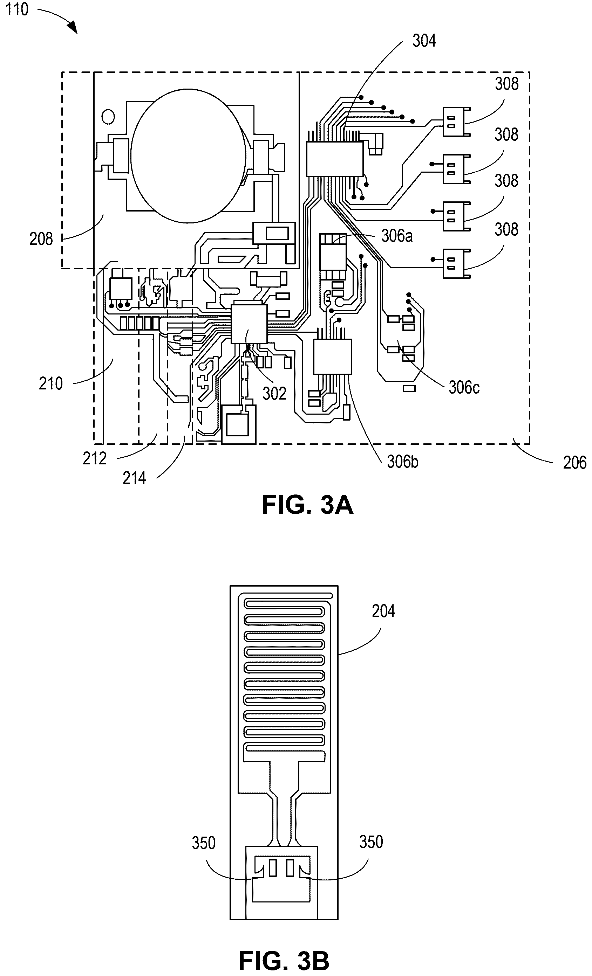

FIG. 3A is a schematic illustration of an example of at least a portion of the corrosion detection sensor 110 of FIGS. 1 and 2. In some examples, including the example of FIG. 3A, the power supply 208 is a cell battery. The power supply 208 powers the 6 axis IMU sensor 210, the light sensor 212, the environment sensors 214, and the impedance analyzer circuitry 206.

In some examples, including the example of FIG. 3A, the impedance analyzer circuitry 206 includes an example microcontroller 302. The microcontroller 302 may include, for example, a Bluetooth low energy microcontroller. In some examples, the impedance analyzer circuitry 206 also includes an example multiplexer (MUX) 304, one or more example reference, buffer, and/or conditioning circuits 306a-c, and one or more examples sensor connectors 308. In some examples, including the illustrated example, there are a reference circuit 306a, an impedance chipset 306b, reference calibration channels 306c, and four sensor connectors 308. The MUX 304, the reference circuits 306, and the microcontroller 302 form part of an electrochemical impedance spectroscopy (EIS) chipset that is used to calculate EIS on the corrosion detection sensor 110 based on impedance data received through the sensor connectors 308 as a function of frequency.

In some examples, firmware of the microcontroller 302 can be programmed to change recording times, mux operations, and/or measurement cycle frequency. Changing the mux operation can allow multiple sensors placed at different places on one or more of the substrates 100 to record and report data. Larger combinations of sensors use power to account for increased data sets, and potential changes to the potentiostat settings. In some examples, the number of sensors operating per device and the number of devices operating on and reporting from a substrate are balanced based on power consumption and/or power availability.

In some examples, the remote device 200 forms a recording network that can be scaled for power and network management to account for an array of individual IoT devices that record and report sensor data.

The sensor connectors 308 are coupled to respective impedance sensors 204. FIG. 3B is a schematic illustration of an example of the impedance sensor 204 of the corrosion detection sensor 110. In some examples, including the example of FIG. 3B, the impedance sensor 204 includes two ports 350. Some other examples may include other numbers of ports such as, for example, four ports. In some examples, the impedance sensor 204 includes printed gold EIS sensor on Kapton. Kapton is a polyimide film used in flexible printed circuits. In some examples, the impedance sensor 204 includes silver printed sensors on Kapton. In some examples, the Kapton is 0.025mm or 1 mm thick. In some examples, the impedance sensor 204 is 10 mm long and 6 mm wide, exclusive of the ports 350. In some examples with printed sensors, the additive nature of printing allows for printing on a flexible substrate and/or on non-planar surfaces. In some examples, the impedance sensor 204 is connectorized or has a direct wire attachment. In some other examples the impedance sensor 204 is patterned directly next to the remaining electronic components. In some examples, use a wide variety of different EIS electrode sensors for one or more impedance sensors 204 in the system can save costs, improve overall system performance, facilitate manufacturability, and enable the re-use of IoT devices (e.g., the remote device 200) when one or more of the impedance sensors 204 is degraded or sensor location is changed. In some examples, the impedance sensor 204 is distal to the connectors 308. For example, the impedance sensor 204 may be wired to one of the connectors 308 via a twelve inch (30.5 cm) long cable. In some examples, the impedance sensor 204 may be placed inside joints, in cracks, between materials or surfaces, under materials (e.g., paint, sealant, CIC, lubricant) or surfaces, etc. In some examples, there are a network of impedance sensors 204 coupled to the connectors 308 to monitor or probe a larger area than monitored with a single impedance sensor 204.

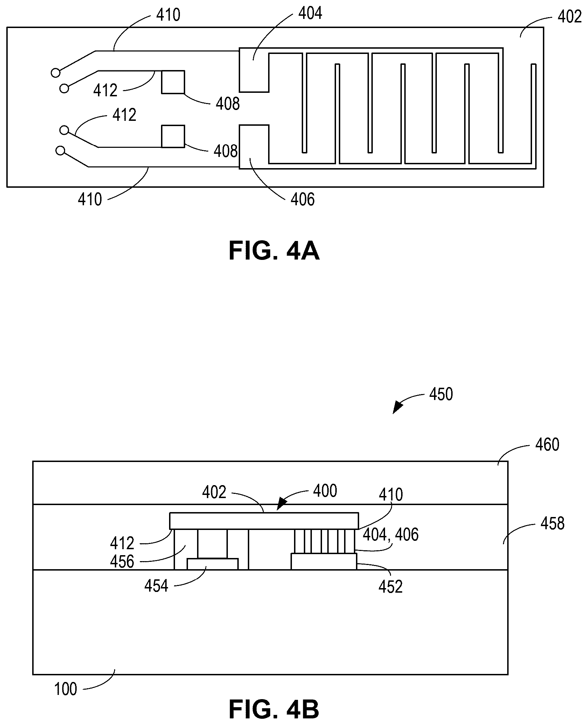

FIG. 4A is a schematic top view of an example IDE 400. The IDE includes a base 402. In some examples, the base 402 includes polyethylene terephthalate (PET). In some examples, including the example of FIG. 4A, the IDE 400 includes a first sensor 0 and a second sensor 406. The first sensor 404 and the second sensor 406 may be used with the impedance sensors 204. The IDE 400 also includes example metallic plate or ink 408. The metallic plate or ink 408 is to be coupled to the substrate 100. Leads or wires 410 from the first sensor 404 and the second sensor 406 and leads or wires 412 from the metallic plate or ink 408 transmit data from the substrate 100 for use in corrosion detection.

FIG. 4B is a schematic side view of another example impedance sensor 450 incorporating the example IDE 400 of FIG. 4A and coupled to the substrate 100. The impedance sensor 450 may implement the impedance sensor 204 of FIGS. 2 and 3B. In some examples, including the illustrated example, the impedance sensor 450 includes an example non-conductive porous material 452 and an example conductive tape 454 via which the impedance sensor 450 is coupled to the substrate 100.

In some examples, the impedance sensor 450 includes an example sealant 456. In some examples, the sealant 456 includes an adhesive sealant. In some examples, the sealant 456 includes a marine sealant. The sealant 456 prevents galvanic corrosion. In some examples, the impedance sensor 450 also includes an example first layer or primer layer 458 and an example second layer or top coat 460. The primer layer 458 and the top coat 460 protect the IDE 400. In some examples, one or more of the primer layer 458 and/or the top coat 460 include paint.

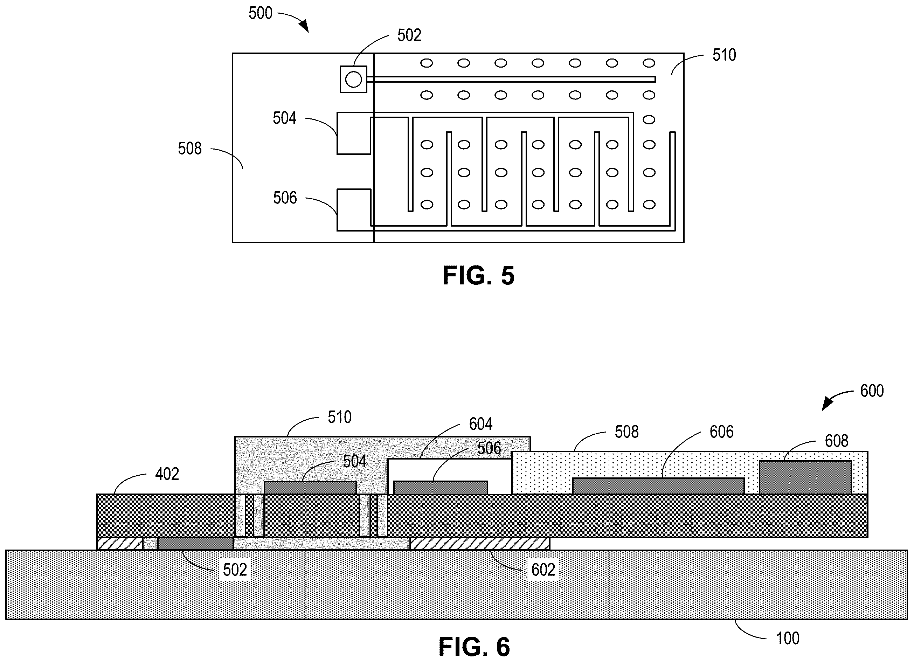

FIG. 5 is a schematic top view of another example impedance sensor 500. The impedance sensor 500 may implement the impedance sensor 204 of FIGS. 2 and 3B. The impedance sensor 500 includes three example electrodes including an anode 502, a cathode 504, and a reference electrode 500 to measure impedance of the substrate 100. The impedance sensor 500 also includes an example encapsulant 508. The encapsulant 508 protects the electrical components from the environment and from corrosion. In some examples, the encapsulant includes acrylic silicone. In some examples the encapsulant includes a potting compound, epoxy, etc. The impedance sensor 500 also includes an example electrolyte 510. The electrolyte 510 facilitates ion transport between the cathode 504 and the anode 502. In some examples, the electrolyte 510 includes a calcium gel, potassium glycerol, sodium chloride, sodium hydroxide, potassium chloride, potassium hydroxide, other electrolyte and/or combination of electrolytes.

In some examples, the impedance sensor 500 is compatible with a 5 electrode EIS detection unit. In some examples, including this example, input current ($I_{in}$), output current ($I_{out}$), input voltage ($V_{in}$), and output voltage ($V_{out}$) can be electrically detected through I V control switches to generate a 5-sensor electrode. The number of sensors available for use with an IoT device (e.g., the remote device 200) is based on the electrical leads at any part of the circuit between the potentiostat, MUX, and sensor.

FIG. 6 is a schematic side view of another example corrosion detection sensor 600. The corrosion detection sensor 600 may implement the corrosion detection sensor 110 of FIGS. 1, 2, and 3B. The corrosion detection sensor 600 incorporates elements of the IDE and/or the impedance sensor 500. In addition, the corrosion detection sensor 600 includes an example adhesive 602 to couple the corrosion detection sensor 600 to the substrate 100. In some examples, the adhesive 602 is patterned. In some examples, the corrosion detection sensor 600 includes adhesive 602 on both sides to couple the corrosion detection sensor 600 between substrates or materials.

The corrosion detection sensor 600 also includes an example layer or top coat 604 to separate the reference electrode 506 from the electrolyte 510. In addition, the corrosion detection sensor 600 includes example control electronics 606. In some examples, the control electronics 606 implement the impedance analyzer circuitry 206. The corrosion detection sensor 600 also include an example antenna 608. In some example, the antenna 608 implements the example communication circuitry 202 of the corrosion detection sensor 110. The antenna 608 can be used to communicate data to the remote device 200.

FIG. 2 is a block diagram of an example of the corrosion detection sensor 110 of FIG. 1 and the remote device 200 to detect corrosion of the substrate 100. The corrosion detection sensor 110 and/or the remote device 200 of FIG. 2 may be instantiated (e.g., creating an instance of, bring into being for any length of time, materialize, implement, etc.) by programmable circuitry such as a Central Processor Unit (CPU) executing first instructions. Additionally or alternatively, the corrosion detection sensor 110 and/or the remote device 200 of FIG. 2 may be instantiated (e.g., creating an instance of, bring into being for any length of time, materialize, implement, etc.) by (i) an Application Specific Integrated Circuit (ASIC), (ii) a Field Programmable Gate Array (FPGA), and/or (iii) other type of microcontroller structured and/or configured in response to execution of second instructions to perform operations corresponding to the first instructions. It should be understood that some or all of the circuitry of FIG. 2 may, thus, be instantiated at the same or different times. Some or all of the circuitry of FIG. 2 may be instantiated, for example, in one or more threads executing concurrently on hardware and/or in series on hardware. Moreover, in some examples, some or all of the circuitry of FIG. 2 may be implemented by microprocessor circuitry executing instructions and/or FPGA circuitry performing operations to implement one or more virtual machines and/or containers.

While an example manner of implementing the corrosion detection sensor 110 and/or the remote device 200 is illustrated in FIG. 2, one or more of the elements, processes, and/or devices illustrated in FIG. 2 may be combined, divided, re-arranged, omitted, eliminated, and/or implemented in any other way. Further, the example communication circuitry 202, the impedance analyzer circuitry 205, the example communication circuitry 220, and/or the example corrosion analyzer circuitry 222 of FIG. 2, may be implemented by hardware alone or by hardware in combination with software and/or firmware. Thus, for example, any of the example communication circuitry 202, the impedance analyzer circuitry 205, the example communication circuitry 220, and/or the example corrosion analyzer circuitry 222, could be implemented by programmable circuitry in combination with machine readable instructions (e.g., firmware or software), processor circuitry, analog circuit(s), digital circuit(s), logic circuit(s), programmable processor(s), programmable microcontroller(s), graphics processing unit(s) (GPU(s)), digital signal processor(s) (DSP(s)), ASIC(s), programmable logic device(s) (PLD(s)), and/or field programmable logic device(s) (FPLD(s)) such as FPGAs. Further still, the example corrosion detection sensor 110 and/or the remote device 200 of FIG. 2 may include one or more elements, processes, and/or devices in addition to, or instead of, those illustrated in FIG. 2 and/or may include more than one of any or all of the illustrated elements, processes and devices.

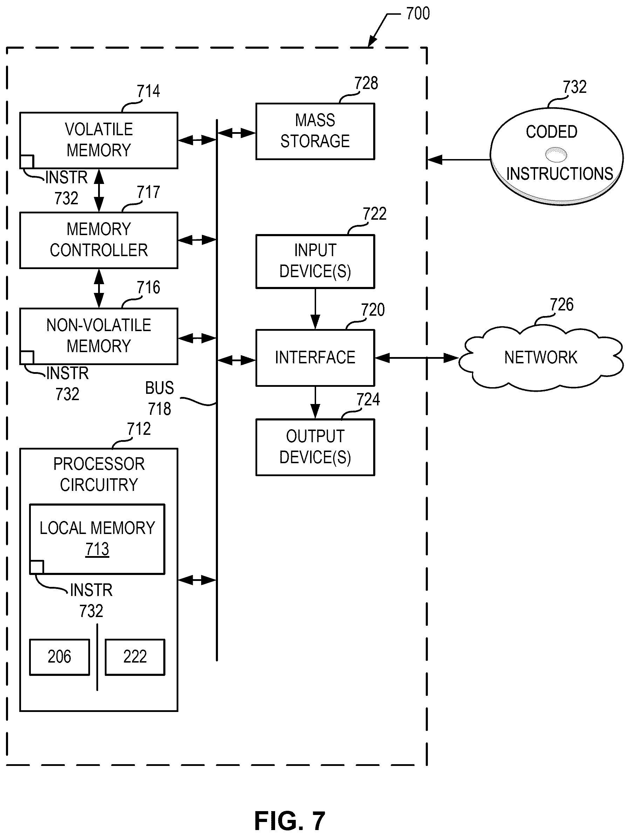

Machine readable instructions may be executed by programmable circuitry to implement and/or instantiate the corrosion detection sensor 110 and/or the remote device 200 of FIG. 2. The machine readable instructions may be one or more executable programs or portion(s) of one or more executable programs for execution by programmable circuitry such as the programmable circuitry 712 shown in the example processor platform 700 discussed below in connection with FIG. 7. In some examples, the machine readable instructions cause an operation, a task, etc., to be carried out and/or performed in an automated manner in the real world. As used herein, "automated" means without human involvement.

The instructions (e.g., software and/or firmware) may be stored on one or more non-transitory computer readable and/or machine readable storage medium such as cache memory, a magnetic-storage device or disk (e.g., a floppy disk, a Hard Disk Drive (HDD), etc.), an optical-storage device or disk (e.g., a Blu-ray disk, a Compact Disk (CD), a Digital Versatile Disk (DVD), etc.), a Redundant Array of Independent Disks (RAID), a register, ROM, a solid-state drive (SSD), SSD memory, non-volatile memory (e.g., electrically erasable programmable read-only memory (EEPROM), flash memory, etc.), volatile memory (e.g., Random Access Memory (RAM) of any type, etc.), and/or any other storage device or storage disk. The instructions of the non-transitory computer readable and/or machine readable medium may program and/or be executed by programmable circuitry located in one or more hardware devices, but the entire program and/or parts thereof could alternatively be executed and/or instantiated by one or more hardware devices other than the programmable circuitry and/or embodied in dedicated hardware. The machine readable instructions may be distributed across multiple hardware devices and/or executed by two or more hardware devices (e.g., a server and a client hardware device). For example, the client hardware device may be implemented by an endpoint client hardware device (e.g., a hardware device associated with a human and/or machine user) or an intermediate client hardware device gateway (e.g., a radio access network (RAN)) that may facilitate communication between a server and an endpoint client hardware device. Similarly, the non-transitory computer readable storage medium may include one or more mediums.

Additionally or alternatively, the instructions may be implemented by one or more hardware circuits (e.g., processor circuitry, discrete and/or integrated analog and/or digital circuitry, an FPGA, an ASIC, a comparator, an operational-amplifier (op-amp), a logic circuit, etc.) structured to perform the corresponding operation without executing software or firmware. The programmable circuitry may be distributed in different network locations and/or local to one or more hardware devices (e.g., a single-core processor (e.g., a single core CPU), a multi-core processor (e.g., a multi-core CPU, an XPU, etc.)). For example, the programmable circuitry may be a CPU and/or an FPGA located in the same package (e.g., the same integrated circuit (IC) package or in two or more separate housings), one or more processors in a single machine, multiple processors distributed across multiple servers of a server rack, multiple processors distributed across one or more server racks, etc., and/or any combination(s) thereof.

The machine readable instructions described herein may be stored in one or more of a compressed format, an encrypted format, a fragmented format, a compiled format, an executable format, a packaged format, etc. Machine readable instructions as described herein may be stored as data (e.g., computer-readable data, machine-readable data, one or more bits (e.g., one or more computer-readable bits, one or more machine-readable bits, etc.), a bitstream (e.g., a computer-readable bitstream, a machine-readable bitstream, etc.), etc.) or a data structure (e.g., as portion(s) of instructions, code, representations of code, etc.) that may be utilized to create, manufacture, and/or produce machine executable instructions. For example, the machine readable instructions may be fragmented and stored on one or more storage devices, disks and/or computing devices (e.g., servers) located at the same or different locations of a network or collection of networks (e.g., in the cloud, in edge devices, etc.). The machine readable instructions may require one or more of installation, modification, adaptation, updating, combining, supplementing, configuring, decryption, decompression, unpacking, distribution, reassignment, compilation, etc., in order to make them directly readable, interpretable, and/or executable by a computing device and/or other machine. For example, the machine readable instructions may be stored in multiple parts, which are individually compressed, encrypted, and/or stored on separate computing devices, wherein the parts when decrypted, decompressed, and/or combined form a set of computer-executable and/or machine executable instructions that implement one or more functions and/or operations that may together form a program such as that described herein.

In some other examples, the machine readable instructions may be stored in a state in which they may be read by programmable circuitry, but require addition of a library (e.g., a dynamic link library (DLL)), a software development kit (SDK), an application programming interface (API), etc., in order to execute the machine-readable instructions on a particular computing device or other device. In some other examples, the machine readable instructions may need to be configured (e.g., settings stored, data input, network addresses recorded, etc.) before the machine readable instructions and/or the corresponding program(s) can be executed in whole or in part. Thus, machine readable, computer readable and/or machine readable media, as used herein, may include instructions and/or program(s) regardless of the particular format or state of the machine readable instructions and/or program(s).

The machine readable instructions described herein can be represented by any past, present, or future instruction language, scripting language, programming language, etc. For example, the machine readable instructions may be represented using any of the following languages: C, C++, Java, C#, Perl, Python, JavaScript, HyperText Markup Language (HTML), Structured Query Language (SQL), Swift, etc.

As mentioned above, the example executable instructions (e.g., computer readable and/or machine readable instructions) may be stored on one or more non-transitory computer readable and/or machine readable media. As used herein, the terms non-transitory computer readable medium, non-transitory computer readable storage medium, non-transitory machine readable medium, and/or non-transitory machine readable storage medium are expressly defined to include any type of computer readable storage device and/or storage disk and to exclude propagating signals and to exclude transmission media. Examples of such non-transitory computer readable medium, non-transitory computer readable storage medium, non-transitory machine readable medium, and/or non-transitory machine readable storage medium include optical storage devices, magnetic storage devices, an HDD, a flash memory, a read-only memory (ROM), a CD, a DVD, a cache, a RAM of any type, a register, and/or any other storage device or storage disk in which information is stored for any duration (e.g., for extended time periods, permanently, for brief instances, for temporarily buffering, and/or for caching of the information). As used herein, the terms "non-transitory computer readable storage device" and "non-transitory machine readable storage device" are defined to include any physical (mechanical, magnetic and/or electrical) hardware to retain information for a time period, but to exclude propagating signals and to exclude transmission media. Examples of non-transitory computer readable storage devices and/ or non-transitory machine readable storage devices include random access memory of any type, read only memory of any type, solid state memory, flash memory, optical discs, magnetic disks, disk drives, and/or redundant array of independent disks (RAID) systems. As used herein, the term "device" refers to physical structure such as mechanical and/or electrical equipment, hardware, and/or circuitry that may or may not be configured by computer readable instructions, machine readable instructions, etc., and/or manufactured to execute computer-readable instructions, machine-readable instructions, etc.

FIG. 7 is a block diagram of an example programmable circuitry platform 700 structured to execute and/or instantiate the example machine-readable instructions to implement the corrosion detection sensor 110 and/or the remote device 200 of FIG. 2. The programmable circuitry platform 700 can be, for example, a server, a personal computer, a workstation, a self-learning machine (e.g., a neural network), a mobile device (e.g., a cell phone, a smart phone, a tablet such as an iPad™), an Internet appliance, or any other type of computing and/or electronic device.

The programmable circuitry platform 700 of the illustrated example includes programmable circuitry 712. The programmable circuitry 712 of the illustrated example is hardware. For example, the programmable circuitry 712 can be implemented by one or more integrated circuits, logic circuits, FPGAs, microprocessors, CPUs, GPUs, DSPs, and/or microcontrollers from any desired family or manufacturer. The programmable circuitry 712 may be implemented by one or more semiconductor based (e.g., silicon based) devices. In some examples, the programmable circuitry platform 700 implements the corrosion detection sensor 110 and the programmable circuitry 712 implements the impedance analyzer circuitry 206. In some examples, the programmable circuitry platform 700 implements the remote device 200 and the programmable circuitry 712 implements the corrosion analyzer circuitry 222.

The programmable circuitry 712 of the illustrated example includes a local memory 713 (e.g., a cache, registers, etc.). The programmable circuitry 712 of the illustrated example is in communication with main memory 714, 716, which includes a volatile memory 714 and a non-volatile memory 716, by a bus 718. The volatile memory 714 may be implemented by Synchronous Dynamic Random Access Memory (SDRAM), Dynamic Random Access Memory (DRAM), RAMBUS® Dynamic Random Access Memory (RDRAM®), and/or any other type of RAM device. The non-volatile memory 716 may be implemented by flash memory and/or any other desired type of memory device. Access to the main memory 714, 716 of the illustrated example is controlled by a memory controller 717. In some examples, the memory controller 717 may be implemented by one or more integrated circuits, logic circuits, microcontrollers from any desired family or manufacturer, or any other type of circuitry to manage the flow of data going to and from the main memory 714, 716.

The programmable circuitry platform 700 of the illustrated example also includes interface circuitry 720. The interface circuitry 720 may be implemented by hardware in accordance with any type of interface standard, such as an Ethernet interface, a universal serial bus (USB) interface, a Bluetooth® interface, a near field communication (NFC) interface, a Peripheral Component Interconnect (PCI) interface, and/or a Peripheral Component Interconnect Express (PCIe) interface.

In some examples, including the illustrated example, one or more input devices 722 are connected to the interface circuitry 720. The input device(s) 722 permit(s) a user (e.g., a human user, a machine user, etc.) to enter data and/or commands into the programmable circuitry 712. The input device(s) 722 can be implemented by, for example, an audio sensor, a microphone, a camera (still or video), a keyboard, a button, a mouse, a touchscreen, a trackpad, a trackball, an isopoint device, and/or a voice recognition system.

One or more output devices 724 are also connected to the interface circuitry 720 of the illustrated example. The output device(s) 724 can be implemented, for example, by display devices (e.g., a light emitting diode (LED), an organic light emitting diode (OLED), a liquid crystal display (LCD), a cathode ray tube (CRT) display, an in-place switching (IPS) display, a touchscreen, etc.), a tactile output device, a printer, and/or speaker. The interface circuitry 720 of the illustrated example, thus, typically includes a graphics driver card, a graphics driver chip, and/or graphics processor circuitry such as a GPU.

The interface circuitry 720 of the illustrated example also includes a communication device such as a transmitter, a receiver, a transceiver, a modem, a residential gateway, a wireless access point, and/or a network interface to facilitate exchange of data with external machines (e.g., computing devices of any kind) by a network 726. The communication can be by, for example, an Ethernet connection, a digital subscriber line (DSL) connection, a telephone line connection, a coaxial cable system, a satellite system, a beyond-line-of-sight wireless system, a line-of-sight wireless system, a cellular telephone system, an optical connection, etc.

The programmable circuitry platform 700 of the illustrated example also includes one or more mass storage discs or devices 728 to store firmware, software, and/or data. Examples of such mass storage discs or devices 728 include magnetic storage devices (e.g., floppy disk, drives, HDDs, etc.), optical storage devices (e.g., Blu-ray disks, CDs, DVDs, etc.), RAID systems, and/or solid-state storage discs or devices such as flash memory devices and/or SSDs.

The machine readable instructions 732, which may be implemented by the machine readable instructions of FIGS. 7, may be stored in the mass storage device 728, in the volatile memory 714, in the non-volatile memory 716, and/or on at least one non-transitory computer readable storage medium such as a CD or DVD which may be removable.

"Including" and "comprising" (and all forms and tenses thereof) are used herein to be open ended terms. Thus, whenever a claim employs any form of "include" or "comprise" (e.g., comprises, includes, comprising, including, having, etc.) as a preamble or within a claim recitation of any kind, it is to be understood that additional elements, terms, etc., may be present without falling outside the scope of the corresponding claim or recitation. As used herein, when the phrase "at least" is used as the transition term in, for example, a preamble of a claim, it is open-ended in the same manner as the term "comprising" and "including" are open ended. The term "and/or" when used, for example, in a form such as A, B, and/or C refers to any combination or subset of A, B, C such as (1) A alone, (2) B alone, (3) C alone, (4) A with B, (5) A with C, (6) B with C, or (7) A with B and with C. As used herein in the context of describing structures, components, items, objects and/or things, the phrase "at least one of A and B" is intended to refer to examples including any of (1) at least one A, (2) at least one B, or (3) at least one A and at least one B. Similarly, as used herein in the context of describing structures, components, items, objects and/or things, the phrase "at least one of A or B" is intended to refer to examples including any of (1) at least one A, (2) at least one B, or (3) at least one A and at least one B. As used herein in the context of describing the performance or execution of processes, instructions, actions, activities, etc., the phrase "at least one of A and B" is intended to refer to examples including any of (1) at least one A, (2) at least one B, or (3) at least one A and at least one B. Similarly, as used herein in the context of describing the performance or execution of processes, instructions, actions, activities, etc., the phrase "at least one of A or B" is intended to refer to examples including any of (1) at least one A, (2) at least one B, or (3) at least one A and at least one B.

As used herein, singular references (e.g., "a", "an", "first", "second", etc.) do not exclude a plurality. The term "a" or "an" object, as used herein, refers to one or more of that object. The terms "a" (or "an"), "one or more", and "at least one" are used interchangeably herein. Furthermore, although individually listed, a plurality of means, elements, or actions may be implemented by, e.g., the same entity or object. Additionally, although individual features may be included in different examples or claims, these may possibly be combined, and the inclusion in different examples or claims does not imply that a combination of features is not feasible and/or advantageous.

Unless specifically stated otherwise, descriptors such as "first," "second," "third," etc., are used herein without imputing or otherwise indicating any meaning of priority, physical order, arrangement in a list, and/or ordering in any way, but are merely used as labels and/or arbitrary names to distinguish elements for ease of understanding the disclosed examples. In some examples, the descriptor "first" may be used to refer to an element in the detailed description, while the same element may be referred to in a claim with a different descriptor such as "second" or "third." In such instances, it should be understood that such descriptors are used merely for identifying those elements distinctly within the context of the discussion (e.g., within a claim) in which the elements might, for example, otherwise share a same name.

As used herein, "approximately" and "about" modify their subjects/values to recognize the potential presence of variations that occur in real world applications. For example, "approximately" and "about" may modify dimensions that may not be exact due to manufacturing tolerances and/or other real world imperfections as will be understood by persons of ordinary skill in the art. For example, "approximately" and "about" may indicate such dimensions may be within a tolerance range of +/- 10% unless otherwise specified herein.

As used herein "substantially real time" refers to occurrence in a near instantaneous manner recognizing there may be real world delays for computing time, transmission, etc. Thus, unless otherwise specified, "substantially real time" refers to real time + 1 second.

As used herein, the phrase "in communication," including variations thereof, encompasses direct communication and/or indirect communication through one or more intermediary components, and does not require direct physical (e.g., wired) communication and/or constant communication, but rather additionally includes selective communication at periodic intervals, scheduled intervals, aperiodic intervals, and/or one-time events.

As used herein, "programmable circuitry" is defined to include (i) one or more special purpose electrical circuits (e.g., an application specific circuit (ASIC)) structured to perform specific operation(s) and including one or more semiconductor-based logic devices (e.g., electrical hardware implemented by one or more transistors), and/or (ii) one or more general purpose semiconductor-based electrical circuits programmable with instructions to perform specific functions(s) and/or operation(s) and including one or more semiconductor-based logic devices (e.g., electrical hardware implemented by one or more transistors). Examples of programmable circuitry include programmable microprocessors such as Central Processor Units (CPUs) that may execute first instructions to perform one or more operations and/or functions, Field Programmable Gate Arrays (FPGAs) that may be programmed with second instructions to cause configuration and/or structuring of the FPGAs to instantiate one or more operations and/or functions corresponding to the first instructions, Graphics Processor Units (GPUs) that may execute first instructions to perform one or more operations and/or functions, Digital Signal Processors (DSPs) that may execute first instructions to perform one or more operations and/or functions, XPUs, Network Processing Units (NPUs) one or more microcontrollers that may execute first instructions to perform one or more operations and/or functions and/or integrated circuits such as Application Specific Integrated Circuits (ASICs). For example, an XPU may be implemented by a heterogeneous computing system including multiple types of programmable circuitry (e.g., one or more FPGAs, one or more CPUs, one or more GPUs, one or more NPUs, one or more DSPs, etc., and/or any combination(s) thereof), and orchestration technology (e.g., application programming interface(s) (API(s)) that may assign computing task(s) to whichever one(s) of the multiple types of programmable circuitry is/are suited and available to perform the computing task(s).

As used herein integrated circuit/circuitry is defined as one or more semiconductor packages containing one or more circuit elements such as transistors, capacitors, inductors, resistors, current paths, diodes, etc. For example an integrated circuit may be implemented as one or more of an ASIC, an FPGA, a chip, a microchip, programmable circuitry, a semiconductor substrate coupling multiple circuit elements, a system on chip (SoC), etc.

From the foregoing, it will be appreciated that example systems, apparatus, articles of manufacture, and methods have been disclosed that can monitor corrosion at regions, ROIs, and/or portions of substrates locally instead of through proxy electrodes that are distal to the ROI. Examples disclosed herein includes impedance sensors that can be independently placed from a remote device such as an IoT device where data related to corrosion is analyzed. These examples allow for targeted measurements in hard-to-reach areas or optimized spatial placement for comprehensive data collection. These examples also include corrosion monitoring and detection device that are of compact size and low power consumption. Examples disclose herein study impedance of substrates and other variables including environmental variables for a comprehensive assessment of conditions related to corrosion development. Examples disclosed herein enable accurate evaluation and prediction of corrosion, leading to improved maintenance strategies and increased operational efficiency in many environments including, for example, aerospace environments.

Example systems, apparatus, articles of manufacture, and methods for corrosion detection are disclosed.

Example 1 includes an apparatus that includes an adhesive to removably couple the apparatus directly to a region of a substrate; a first sensor to measure a first parameter at the region; a second sensor to measure a second parameter at the region, the first parameter and the second parameter being factors of corrosion at the region; and a power supply to provide power to the first sensor and the second sensor.

Example 2 includes the apparatus of Example 1, wherein the region includes a crevice in the substrate.

Example 3 includes the apparatus of any of Examples 1-2, wherein the substrate is a portion of a vehicle.

Example 4 includes the apparatus of any of Examples 1-2, wherein the substrate is a portion of at least one of a structure with a static platform or an oscillating platform.

Example 5 includes the apparatus of any of Examples 1-4, further including: machine-readable instructions; and at least one processor circuit to be programmed by the machine-readable instructions to wirelessly communicate the first parameter and the second parameter to a device remote from the region to determine a level of corrosion of the region based on the first parameter and the second parameter.

Example 6 includes the apparatus of any of Examples 1-5, wherein the first sensor includes electrodes and the first parameter is impedance.

Example 7 includes the apparatus of Example 6, wherein second sensor includes a light sensor, and the second parameter is a level of ultraviolet light exposure.

Example 8 includes the apparatus of any of Examples 6-7, wherein the second sensor includes an inertial measurement unit, and the second parameter includes one or more of a linear acceleration, a rotational rate, or a force.

Example 9 includes the apparatus of any of Examples 1-8, wherein the region is under at least one of a layer of paint sealant, lubricant, or corrosion-inhibiting compound.

Example 10 includes an Internet of Things (IoT) device that includes an impedance sensor to measure impedance of a substrate; machine-readable instructions; at least one processor circuit to be programmed by the machine-readable instructions to determine the impedance of the substrate from the impedance sensor at multiple frequencies; a power supply to power the impedance sensor and the at least one processor circuit; and an adhesive to

couple the IoT device to the substrate.

Example 11 includes the IoT device of Example 10, wherein the at least one processor is to determine the impedance of the substrate over time.

Example 12 includes the IoT device of any of Examples 10-11, wherein the at least one processor is to wirelessly communicate data related to the impedance of the substate to a remote device.

Example 13 includes the IoT device of any of Examples 10-12, further including an environmental sensor to measure an environmental parameter of the substrate.

Example 14 includes the IoT device of Example 13, wherein the environmental parameter is a percentage of a gas in the environment of the substrate.

Example 15 includes an aircraft that includes a corrosion detection sensor including: an impedance sensor to measure impedance at a portion of a substrate of the aircraft; first machine-readable instructions; and first programmable circuitry to be programmed by the first machine-readable instructions to wirelessly communicate data related to the impedance; and a device remote from the corrosion detection sensor, the device including: second machine-readable instructions; and second programmable circuitry to be programmed by the second machine-readable instructions to: wirelessly access the data related to the impedance; and determine a level of corrosion of the portion based on the data related to the impedance.

Example 16 includes the aircraft of Example 15, wherein the corrosion detection sensor is a first corrosion detection sensor, the impedance sensor is a first impedance sensor, the portion is a first portion, and the substrate is a first substrate, the aircraft further including: a second corrosion detection sensor including: a second impedance sensor to measure impedance at a second portion of a second substrate of the aircraft, the second substrate different than the first substrate; third machine-readable instructions; and third programmable circuitry to be programmed by the third machine-readable instructions to wirelessly communicate data related to the impedance at the second portion, wherein the device is remote from the second corrosion detection sensor and the second programmable circuitry is configured to: wirelessly access the data related to the impedance at the second portion; and determine a second level of corrosion of the second portion based on the data related to the impedance at the second portion.

Example 17 includes the aircraft of Example 15, wherein the corrosion detection sensor is a first corrosion detection sensor, the impedance sensor is a first impedance sensor, the portion is a first portion, the aircraft further including: a second corrosion detection sensor including: a second impedance sensor to measure impedance at a second portion of the substrate; third machine-readable instructions; and third programmable circuitry to be programmed by the third machine-readable instructions to wirelessly communicate data related to the impedance at the second portion, wherein the device is remote from the second corrosion detection sensor and the second programmable circuitry is configured to: wirelessly access the data related to the impedance at the second portion; and determine the level of corrosion of the substrate based on the data related to the impedance at the first portion and the data related to the impedance at the second portion.

Example 18 includes the aircraft of any of Examples 15-17, wherein the portion is a lap joint.

Example 19 includes the aircraft of any of Examples 15-18, wherein the portion is at least one of at, near, between, or under at least one of a bolt or a washer.

Example 20 includes the aircraft of any of Examples 15-19, wherein the first programmable circuitry is configured to wirelessly communicate the data related to the impedance and the second programmable circuitry is configured to determine the level of corrosion of the portion before, during, and/or after flight.

The following claims are hereby incorporated into this Detailed Description by this reference. Although certain example systems, apparatus, articles of manufacture, and methods have been disclosed herein, the scope of coverage of this patent is not limited thereto. On the contrary, this patent covers all systems, apparatus, articles of manufacture, and methods fairly falling within the scope of the claims of this patent.

**Claims**

1. A corrosion detection sensor (110) comprising:

   an adhesive (602) configured to removably couple the corrosion detection sensor (110) directly to a region of a substrate (100);
   a first sensor (204, 210, 212, 214, 404, 406, 450, 500) for measuring a first parameter at the region;
   a second sensor (204, 210, 212, 214, 404, 406, 450, 500) for measuring a second parameter at the region, the first parameter and the second parameter being factors of corrosion at the region; and
   a power supply (208) to provide power to the first sensor (204, 210, 212, 214, 404, 406, 450, 500) and the second sensor (204, 210, 212, 214, 404, 406, 450, 500).

2. The corrosion detection sensor (110) of claim 1, further including:

at least one processor circuit (202, 206, 712); and
machine-readable instructions (732) that, when executed by the at least one processor circuit (202, 206, 712), cause the at least one processor circuit (202, 206, 712) to wirelessly communicate the first parameter and the second parameter to a device (200) remote from the region for determining a level of corrosion of the region based on the first parameter and the second parameter.

3. The corrosion detection sensor (110) of claim 1 or claim 2, wherein the first sensor (204, 210, 212, 214, 404, 406, 450, 500) includes electrodes (270, 272, 500, 502, 504) and the first parameter is impedance, and optionally, wherein:

the second sensor includes a light sensor (212), and the second parameter is a level of ultraviolet light exposure; and/or
the second sensor includes an inertial measurement unit (210), and the second parameter includes one or more of a linear acceleration, a rotational rate, or a force.

4. The corrosion detection sensor (110) of claim 1, wherein:

the first sensor is an impedance sensor (204, 450, 500) configured to measure impedance of the substrate (100);
the corrosion detection sensor (110) further includes:

at least one processor circuit (202, 206, 712); and
machine-readable instructions (732) that, when executed by the at least one processor circuit (202, 206, 712) cause the at least one processor circuit (202, 206, 712) to determine the impedance of the substrate (100) from the impedance sensor (204, 450, 500) at multiple frequencies; and

the power supply (208) is further configured to power the at least one processor circuit (202, 206, 712).

5. The corrosion detection sensor (110) of claim 4, wherein the at least one processor (202, 206, 712) is configured to determine the impedance of the substrate (100) over time.

6. The corrosion detection sensor (110) of claim 4 or claim 5, wherein the at least one processor (202, 206, 712) is configured to wirelessly communicate data related to the impedance of the substate (100) to a remote device (200).

7. The corrosion detection sensor (110) of any one of claims 4 - 6, further including an environmental sensor (214) for measuring an environmental parameter of the substrate (100), and optionally, wherein the environmental parameter is a percentage of a gas in the environment of the substrate (100).

8. A system comprising:

a substrate (100) comprising a region;
the corrosion detection sensor (110) of any preceding claim; and optionally,
a device (200) remote from the corrosion detection sensor (110), the device configured to determine a level of corrosion of the region based on the first parameter and the second parameter.

9. The system of claim 8, wherein one of:

the substrate (100) is a portion of a vehicle; or
the substrate (100) is a portion of at least one of a structure with a static platform or an oscillating platform, and optionally, wherein the static platform is one of a building, a pipe and a metal object, and wherein the oscillating platform is a bridge.

10. The system of claim 8 or claim 9, wherein one or more of:

the region of the substrate includes a crevice in the substrate (100); and
the region is under at least one of a layer of paint, sealant, lubricant, or corrosion-inhibiting compound.

11. An aircraft comprising:

a first substrate (100);
a first corrosion detection sensor (110) according to any of claims 4 - 7, wherein the impedance sensor (204, 450, 500) of the first corrosion detection sensor (110) is configured to measure the impedance at a portion of the first substrate (100), and wherein the at least one processor circuit (202, 206, 712) of the first corrosion detection sensor (110) is configured to wirelessly communicate data related to the impedance; and
a device (200) remote from the first corrosion detection sensor (110), the device (200) including:

device programmable circuitry (220, 222, 712); and
device machine-readable instructions (732) that, when executed by the device programmable circuitry (220, 222, 712) cause the device programmable circuitry (220, 222, 712) to:

wirelessly access the data related to the impedance; and
determine a level of corrosion of the portion based on the data related to the impedance.

12. The aircraft of claim 11, wherein the at least one processor circuit (202, 206, 712) of the first corrosion detection sensor (110) is configured to wirelessly communicate the data related to the impedance and the device programmable circuitry (220, 222, 712) is configured to determine the level of corrosion of the portion before, during, or after flight.

13. The aircraft of claim 11 or claim 12, wherein one or more of:

the portion is a lap joint;
the portion is at least one of at, near, between, or under at least one of a bolt or a washer; and
the adhesive (602) of the first corrosion detection sensor (110) is configured to removably couple the corrosion detection sensor (110) directly to the portion of the first substrate (100).

14. The aircraft of any of claims 11 - 13, further including a second substrate different than the first substrate (100) and a second corrosion detection sensor (110) according to any of claims 4 - 7, and wherein:

the impedance sensor (204, 450, 500) of the second corrosion detection sensor (110) is configured to measure impedance at a second portion of the second substrate;
the at least one processor circuit (202, 206, 712) of the second corrosion detection sensor (110) is configured to wirelessly communicate data related to the impedance at the second portion; and
the device (200) is remote from the second corrosion detection sensor (110) and the device programmable circuitry (202, 206, 712) is configured to:

wirelessly access the data related to the impedance at the second portion; and
determine a second level of corrosion of the second portion based on the data related to the impedance at the second portion.

15. The aircraft of any of claims 11 - 13, further including a second corrosion detection sensor (110) according to any of claims 4 - 7, and wherein:

the impedance sensor (204, 450, 500) of the second corrosion detection sensor (110) is configured to measure impedance at a second portion of the first substrate (100);
the at least one processor circuit (202, 206, 712) of the second corrosion detection sensor (110) is configured to wirelessly communicate data related to the impedance at the second portion; and
the device (200) is remote from the second corrosion detection sensor (110) and the device programmable circuitry (220, 222, 712) is configured to:

wirelessly access the data related to the impedance at the second portion; and
determine the level of corrosion of the first substrate (100) based on the data related to the impedance at the first portion and the data related to the impedance at the second portion.

**FIG. 1**

EP 4 667 904 A1

**FIG. 2**

EP 4 667 904 A1

**FIG. 3A**

**FIG. 3B**

**FIG. 4A**

**FIG. 4B**

**FIG. 5**

**FIG. 6**

EP 4 667 904 A1

**FIG. 7**

Europäisches
Patentamt

European
Patent Office

Office européen
des brevets

# EUROPEAN SEARCH REPORT

**Application Number**

EP 25 18 0447

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 6 328 878 B1 (DAVIS GUY D [US] ET AL) 11 December 2001 (2001-12-11) * abstract * * column 1 - column 12 * * figures 1-4 * ----- | 1-15 | INV. G01N17/00 |
| A | US 9 518 915 B2 (LUNA INNOVATIONS INC [US]) 13 December 2016 (2016-12-13) * abstract * * column 4 * ----- | 1-15 | |

**TECHNICAL FIELDS
SEARCHED (IPC)**

G01N

The present search report has been drawn up for all claims

| Place of search | Date of completion of the search | Examiner |
|---|---|---|
| The Hague | 21 October 2025 | Appeltant, Lennert |

## ANNEX TO THE EUROPEAN SEARCH REPORT
## ON EUROPEAN PATENT APPLICATION NO.

EP 25 18 0447

This annex lists the patent family members relating to the patent documents cited in the above-mentioned European search report.
The members are as contained in the European Patent Office EDP file on
The European Patent Office is in no way liable for these particulars which are merely given for the purpose of information.

21-10-2025

| Patent document cited in search report | | Publication date | Patent family member(s) | | Publication date |
|---|---|---|---|---|---|
| US 6328878 | B1 | 11-12-2001 | NONE | | |
| US 9518915 | B2 | 13-12-2016 | US | 2015268152 A1 | 24-09-2015 |
| | | | WO | 2014018288 A1 | 30-01-2014 |

EPO FORM P0459

For more details about this annex : see Official Journal of the European Patent Office, No. 12/82